# EUROPEAN PATENT SPECIFICATION

(11) **EP 2 344 621 B1**
(45) Date of publication and mention of the grant of the patent: **06.01.2016**
(21) Application number: 09796429.0
(22) Date of filing: 16.10.2009
(51) Int. Cl.: C12M 1/107

(54) **ANAEROBIC DIGESTER**
ANAEROBER FAULBEHÄLTER
DIGESTEUR ANAÉROBIE

(30) Priority: 17.10.2008 IT BO20080641
(43) Date of publication of application: 20.07.2011
(73) Proprietor: Ambientalia S.R.L., 40050 Dozza (Bologna) (IT)
(72) Inventor: BRESSAN, Loris, I-40026 Imola (IT)
(74) Representative: Firmati, Leonardo
(86) International application number: PCT/IB2009/007152
(87) International publication number: WO 2010/043964

(56) References cited:
- EP-A- 0 023 176
- EP-A- 0 028 053
- DE-U1- 20 203 533
- DE-U1-202005 014 176
- US-A- 4 060 175

## Description

### TECHNICAL FIELD

The present invention relates to an anaerobic digester. In particular, the digester of the present invention is adapted to implement both the anaerobic and aerobic treatment steps for biomasses.

### BACKGROUND ART

The problem of disposal or recycling of waste has been particularly felt for a long time. In particular, organic waste is the most problematic issue because of its high environmental impact.

In this respect, the new European regulations related to the waste disposal indicate the processes of sanitation and composting as particularly valuable solutions.

Composting is a natural form of organic waste disposal known for a long time and by which the compost is obtained, which is a product employed in agriculture as soil improver, fertilizer or growing medium.

In particular, composting is an exothermic dioxide process occurring under controlled conditions and leading to the production of water, carbon dioxide, heat and compost by the action of microorganisms.

Another form of organic waste exploitation is related to the production of biogas by means of anaerobic fermentation. The produced biogas can either be converted into power and/or heat through a cogeneration system, or be directly placed into the gas network.

In order to exploit both the potential of the biogas and that of the compost, the process of recycling the organic waste should provide for two consequential steps: a first step in which organic waste is subjected to an anaerobic treatment for the formation of biogas, and a second step in which it is subjected to an aerobic treatment for the preparation of the compost.

As it may be obvious, the shift from first to second step may imply a series of problems, especially of environmental and logistical nature. Indeed, the transport of waste from anaerobic to aerobic reactor is particularly complex and obviously environmentally hazardous.

Furthermore, the anaerobic digesters commonly in use, such as described in DE 202005014176 U, provide for a remaining overhanging empty space of about 2-3 m once the mass has been accommodated therein, which space is required for operating the shovel during the biomass loading and unloading operations. The presence of such a space involves some drawbacks in the anaerobic digestion step. Indeed, during the anaerobic digestion step, the produced heat and humidity fill the aforementioned overhanging empty space, thus causing a dispersion inside the biomass. In order to obviate this dispersion, a large amount of power should be used especially in order to keep the temperature within the mass.

### DISCLOSURE OF INVENTION

It is the object of the present invention to provide a digester, the technical features of which are such as to overcome the above-described drawbacks while being easily and cost-effectively to be implemented.

The object of the present invention is a digester whose essential features are set forth in claim 1, and whose preferred and/or auxiliary features are set forth in claims 2-5.

### BRIEF DESCRIPTION OF THE DRAWINGS

For a better understanding of the present invention, a preferred embodiment is now described only by way of non-limiting illustrative example, with reference to the accompanying drawings, in which:
- figure 1 is a perspective view with parts in section of the digester object of the present invention in an operating configuration; and
- figure 2 is a perspective view with parts in section of the digester in figure 1 in a further operating configuration.

### BEST MODE FOR CARRYING OUT THE INVENTION

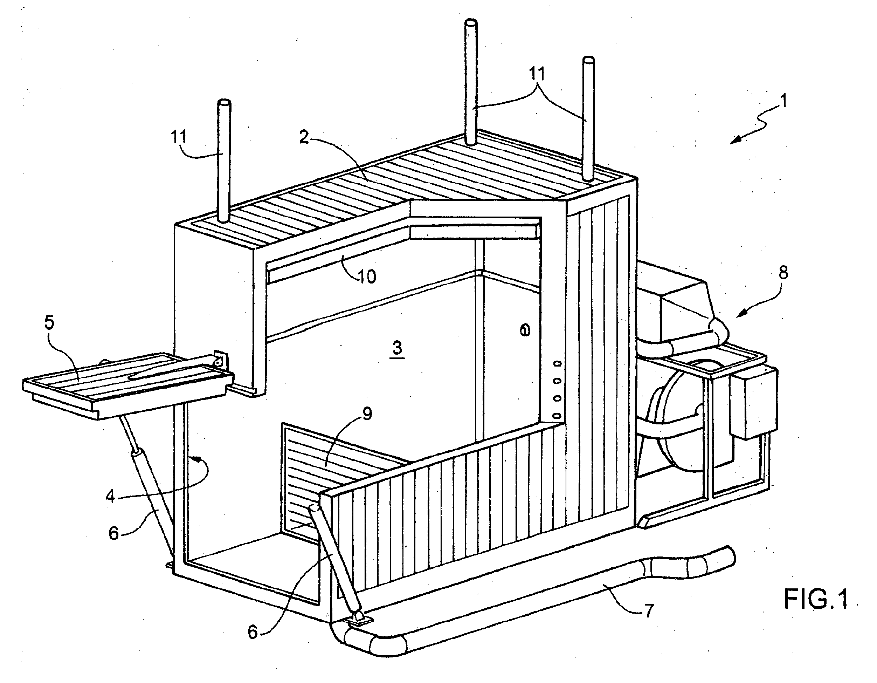

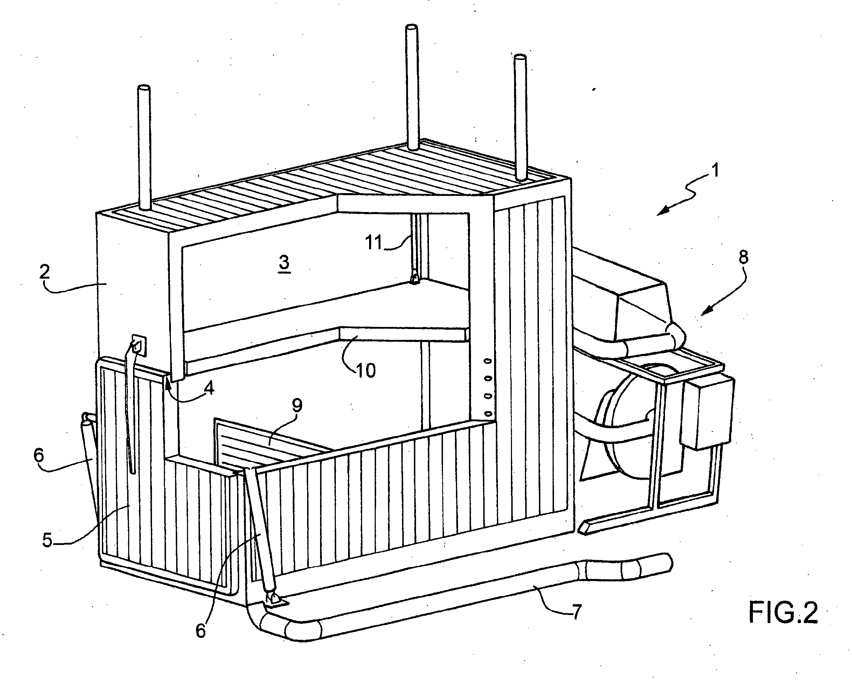

In figures 1 and 2, numeral 1 indicates as a whole the digester object of the present invention. Digester 1 comprises a parallelepiped-shaped containing structure 2 defining a housing chamber 3 therein for the biomass to be treated. In the containing structure 2, a front opening 4 is obtained for the inlet and outlet of the biomass, which can be closed by a door 5 hinged above the containing structure 2 itself and which can be moved by operating means 6, preferably hydraulic means.

Digester 1 comprises a waste pipe 7 for the removal of percolate and a ventilation group 8 responsible for the aeration inside the housing chamber 3 by means of pipes and openings (known and not described) facing each other in the housing chamber 3.

Digester 1 further comprises a containing wall 9 placed inside the housing chamber 3 which is useful for spacing apart the biomass from the front opening 4.

Finally, digester 1 comprises a mobile false ceiling 10 placed inside the housing chamber 3, which can be moved by operating means 11, preferably pneumatic means, between a raised position (figure 1), in which the operation of the mobile shovel for loading and unloading the biomass is allowed, and a lowered position (figure 2) which is useful during the anaerobic treatment step as described below.

In use, starting from the usage configuration shown in figure 1, a shovel (known and not shown) is allowed to perform the loading operations. Once the loading step is complete, door 5 is closed and the mobile false ceiling 10 is lowered, thus implementing the usage configuration shown in figure 2. The mobile false ceiling 10 is lowered, thus leaving a small gap between the same and the biomass to be treated. The anaerobic process is performed in the configuration thus obtained. In this configuration, the known art energy waste is avoided as the overhanging empty space has been reduced due to the possibility of placing a movable false ceiling that can be taken to a lowered position. Once the anaerobic process is complete, the aerobic treatment step is started, without the need for moving the biomass. During the aerobic treatment, the ventilation group 8 is operated. Once the aerobic treatment step is also complete, the false ceiling 10 is taken back to its raised position, and door 5 is opened for letting the shovel in to perform the unloading operations with maximum safety and comfort.

The intake of large amounts of air by the ventilation group 8 for supporting the aerobic treatment step may be used during the loading and unloading step for keeping the suitable environmental conditions for the operators to enter the housing chamber.

As it is apparent from the above, the digester of the present invention allows to perform an anaerobic treatment step without the dispersion of the known art digesters, and therefore without requiring to use external power for ensuring the temperature preservation within the biomass. Furthermore, the digester object of the present invention allows both the anaerobic treatment and the aerobic treatment to be sequentially carried out, without having to move the biomass at all.

## Claims

1. Anaerobic digester (1) comprising a containing structure (2) defining inside it a housing chamber (3) for the biomass to be treated; the containing structure (2) having an opening (4) for the inlet and the outlet of the biomass, which can be closed by a door (5); said digester being **characterized in that** it comprises a mobile false ceiling (10), placed inside the housing chamber (3)**,and operating means (11) by which the mobile false ceiling (10)** can be moved between a raised position and a lowered position.

2. Anaerobic digester (1) according to Claim 1, **characterized in that** said operating means (11) are hydraulic means.

3. Anaerobic digester (1) according to Claim 1 or 2, **characterized in that** it comprises a ventilation group (8) for the aeration inside the housing chamber (3).

4. Anaerobic digester (1) according to Claim 3, **characterized in that** it comprises a waste pipe for the removal of percolate (7).

5. Anaerobic digester (1) according to Claim 4, **characterized in that** it comprises a containing wall (9) placed inside the housing chamber for spacing the biomass from said opening (4).

## Patentansprüche

1. Anaerober Faulbehälter (1), umfassend eine Behälterstruktur (2), definierend im Innenraum eine Aufnahmekammer (3) für die zu behandelnde Biomasse, wobei die Behälterstruktur (2) eine Öffnung (4) für das Zuführen und das Abführen der Biomasse umfasst, die durch eine Tür (5) verschlossen werden kann, wobei der Faulbehälter **dadurch gekennzeichnet ist, dass** er eine bewegliche Zwischendecke (10) umfasst, der in der Aufnahmekammer (3) platziert ist, sowie Betriebsmittel (11), mittels derer die bewegliche Zwischendecke (10) zwischen einer angehobenen und einer gesenkten Position bewegt werden kann.

2. Anaerober Faulbehälter (1) nach Anspruch 1, **dadurch gekennzeichnet, dass** es sich bei den Betriebsmitteln (11) um hydraulische Mittel handelt.

3. Anaerober Faulbehälter (1) nach Anspruch 1 oder 2, **dadurch gekennzeichnet, dass** er eine Lüftereinheit (8) für die Innenbelüftung der Aufnahmekammer (3) umfasst.

4. Anaerober Faulbehälter (1) nach Anspruch 3, **dadurch gekennzeichnet, dass** er eine Abführleitung für die Beseitigung von Sickerflüssigkeit (7) umfasst.

5. Anaerober Faulbehälter (1) nach Anspruch 4, **dadurch gekennzeichnet, dass** er eine Behälterwand (9) umfasst, die in der Aufnahmekammer untergebracht ist, um die Biomasse von der Öffnung (4) zu beabstanden.

## Revendications

1. Digesteur anaérobie (1) comprenant une structure de confinement (2) définissant en son sein une chambre de logement (3) destinée à la biomasse à traiter ; la structure de confinement (2) ayant une ouverture (4) pour l'introduction ou le retrait de la biomasse qui peut être fermée par une porte (5); ledit digesteur étant **caractérisé en ce qu'**il comprend un faux plafond mobile (10), placé à l'intérieur de la chambre de logement (3), et des moyens d'actionnement (11) par lesquels le faux plafond mobile (10) peut être déplacé entre une position relevée et une position abaissée.

2. Digesteur anaérobie (1) selon la revendication 1, **caractérisé en ce que** lesdits moyens d'actionnement (11) sont des moyens hydrauliques.

3. Digesteur anaérobie (1) selon les revendications 1 ou 2, **caractérisé en ce qu'**il comprend un groupe de ventilation (8) destiné à ventiler l'intérieur de la chambre de logement (3).

4. Digesteur anaérobie (1) selon la revendication 3, **caractérisé en ce qu'**il comprend une conduite d'évacuation du lixiviat (7).

5. Digesteur anaérobie (1) selon la revendication 4, **caractérisé en ce qu'**il comprend une cloison de confinement (9), placée à l'intérieur de la chambre de logement, destinée à séparer la biomasse de ladite ouverture (4).
